(19) Europäisches Patentamt
European Patent Office
Office européen des brevets

(11) EP 4 357 330 A1

(12) **EUROPEAN PATENT APPLICATION**
published in accordance with Art. 153(4) EPC

(43) Date of publication:
**24.04.2024 Bulletin 2024/17**

(21) Application number: **22825072.6**

(22) Date of filing: **16.06.2022**

(51) International Patent Classification (IPC):
***C07C 43/205*** $^{(2006.01)}$ ***C08G 8/28*** $^{(2006.01)}$
***C08L 61/12*** $^{(2006.01)}$ ***C09K 3/18*** $^{(2006.01)}$

(52) Cooperative Patent Classification (CPC):
**C07C 43/225; C07C 43/21; C07C 271/44; C08G 10/00; C08G 61/02; C08L 61/12; C08L 65/00; C09D 165/00; C09K 3/18;**
C07C 2603/92; C08G 2261/1412; C08G 2261/1424; C08G 2261/143; C08G 2261/1432; C08G 2261/146;

(Cont.)

(86) International application number:
**PCT/JP2022/024222**

(87) International publication number:
**WO 2022/265084 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.06.2021 JP 2021101149**

(71) Applicants:
- **Kyoto University**
  **Kyoto-shi, Kyoto 606-8501 (JP)**
- **DAIKIN INDUSTRIES, LTD.**
  **Osaka-Shi, Osaka 530-0001 (JP)**

(72) Inventors:
- **OGOSHI, Tomoki**
  **Kyoto-shi, Kyoto 606-8501 (JP)**
- **ONISHI, Katsuto**
  **Kyoto-shi, Kyoto 606-8501 (JP)**
- **YAMAGUCHI, Shuhei**
  **Osaka-shi, Osaka 530-8323 (JP)**
- **SHIOTANI, Yuko**
  **Osaka-shi, Osaka 530-8323 (JP)**
- **KOMORI, Masaji**
  **Osaka-shi, Osaka 530-8323 (JP)**
- **AOYAMA, Hirokazu**
  **Osaka-shi, Osaka 530-8323 (JP)**
- **KISHIKAWA, Yosuke**
  **Osaka-shi, Osaka 530-8323 (JP)**
- **YAMAUCHI, Akiyoshi**
  **Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **FLUORINE-CONTAINING PILLARARENE**

(57) The present invention provides a compound represented by Formula (1A) below wherein the symbols are as defined in the description:

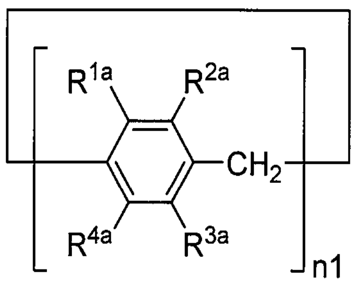

EP 4 357 330 A1

(52) Cooperative Patent Classification (CPC): (Cont.)
C08G 2261/148; C08G 2261/226;
C08G 2261/342; C08G 2261/42

## Description

Technical Field

The present disclosure relates to a fluorinecontaining pillararene.

Background Art

As a cyclic compound, a compound such as a pillararene, a crown ether, a cyclodextrin, a calixarene, or a cucurbituril is known. Among these, a pillararene, which has a symmetrical pillar structure, has attracted attention because of its various properties such as the ability to function as a host molecule that can incorporate a guest molecule (Patent Literature 1).

Citation List

Patent Literature

Patent Literature 1: JP 2015-221893 A

Summary of Invention

Technical Problem

An object of the present disclosure is to provide a novel pillararene having good water repellency, and particularly a novel pillararene containing a fluorine atom.

Solution to Problem

The present disclosure includes the following aspects.

[1] A compound represented by Formula (1A) below:

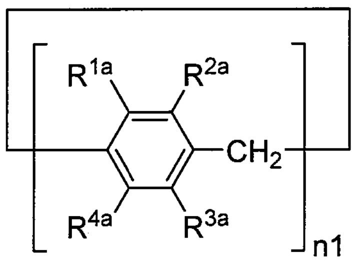

wherein

$R^{1a}$ is each independently at each occurrence a hydrogen atom or an organic group,
$R^{2a}$ is each independently at each occurrence a hydrogen atom or an organic group,
$R^{3a}$ is each independently at each occurrence a hydrogen atom or an organic group,
$R^{4a}$ is each independently at each occurrence a hydrogen atom or an organic group,
provided that at least one of $R^{1a}$, $R^{2a}$, $R^{3a}$, and $R^{4a}$ is an organic group having 2 or more carbon atoms and having a fluoroalkyl group or a fluorophenyl group, and
n1 is an integer of 4 to 20.

[2] The compound according to [1] above, wherein

$R^{1a}$ and $R^{3a}$ are each independently an organic group,
provided that at least one of $R^{la}$ and $R^{3a}$ is an organic group having 2 or more carbon atoms and having a fluoroalkyl group or a fluorophenyl group, or
$R^{2a}$ and $R^{4a}$ are each independently an organic group,

provided that at least one of $R^{2a}$ and $R^{4a}$ is an organic group having 2 or more carbon atoms and having a fluoroalkyl group or a fluorophenyl group.

[3] The compound according to [1] or [2] above, wherein

$R^{1a}$ and $R^{3a}$ are each independently an organic group having 2 or more carbon atoms and having a fluoroalkyl group or a fluorophenyl group, or
$R^{2a}$ and $R^{4a}$ are each independently an organic group having 2 or more carbon atoms and having a fluoroalkyl group or a fluorophenyl group.

[4] The compound according to any one of [1] to [3] above, wherein

$R^{2a}$ and $R^{4a}$ are each a hydrogen atom, or
$R^{1a}$ and $R^{3a}$ are each a hydrogen atom.

[5] The compound according to any one of [1] to [4] above, wherein n1 is an integer of 4 to 6.
[6] The compound according to any one of [1] to [5] above, wherein

the organic group having 2 or more carbon atoms and having a fluoroalkyl group is $-(O_{p1}-R^{11a}{}_{q1})-Rf^a$,
$R^{11a}$ is each independently at each occurrence a $C_{1-10}$ alkylene group or -CONH-,
$Rf^a$ is a $C_{1-10}$ fluoroalkyl group,
p1 is an integer of 0 to 2,
q1 is an integer of 0 to 3, and
in $(O_{p1}-R^{11a}{}_{q1})$, occurrence order of O and $R^{11a}$ is not limited.

[7] The compound according to any one of [1] to [6] above, wherein

the organic group having 2 or more carbon atoms and having a fluoroalkyl group is $-O-R^{11a}-(O-R^{11a})_{r1}-Rf^a$,
$R^{11a}$ is each independently a $C_{1-10}$ alkylene group or -CONH-,
$Rf^a$ is a $C_{1-10}$ fluoroalkyl group, and
r1 is 0 or 1.

[8] The compound according to any one of [1] to [7] above, wherein the fluoroalkyl group is a perfluoroalkyl group.
[9] The compound according to any one of [1] to [8] above, wherein

$R^{1a}$ and $R^{3a}$ are each $-O-R^{11a}-(O-R^{11a})_{r1}-Rf^a$, and $R^{2a}$ and $R^{4a}$ are each a hydrogen atom, or
$R^{2a}$ and $R^{4a}$ are each $-O-R^{11a}-(O-R^{11a})_{r1}-Rf^a$, and $R^{1a}$ and $R^{3a}$ are each a hydrogen atom,
$R^{11a}$ is each independently a $C_{1-10}$ alkylene group or -CONH-,
$Rf^a$ is a $C_{1-10}$ perfluoroalkyl group,
r1 is 0 or 1, and
n1 is an integer of 4 to 6.

[10] The compound according to any one of [1] to [5] above, wherein
the organic group having 2 or more carbon atoms and having a fluorophenyl group is a group represented by the following formula:

$$-(O_{p3}-R^{11p}{}_{q3})-RfP$$

wherein

$R^{11p}$ is each independently at each occurrence a $C_{1-10}$ alkylene group,
RfP is a phenyl group substituted by 1 to 5 fluorine,
p3 is an integer of 0 to 2,
q3 is an integer of 0 to 3, and
in $(O_{p3}-R^{11p}{}_{q3})$, occurrence order of O and $R^{11p}$ is not limited.

[11] The compound according to any one of [1] to [5] above, wherein
the organic group having 2 or more carbon atoms and having a fluorophenyl group is a group represented by the

following formula:

$$-O-R^{11p}-Rf^{p}$$

wherein

$R^{11p}$ is each independently at each occurrence a $C_{1-10}$ alkylene group, and
RfP is a phenyl group substituted with 1 to 5 fluorine atoms.

[12] The compound according to any one of [1] to [5] above, wherein

$R^{1a}$ and $R^{3a}$ are each $-O-R^{11p}-Rf^{p}$, and $R^{2a}$ and $R^{4a}$ are each a hydrogen atom, or
$R^{2a}$ and $R^{4a}$ are $-O-R^{11p}-Rf^{p}$, and $R^{1a}$ and $R^{3a}$ are each a hydrogen atom,
$R^{11p}$ is each independently a $C_{1-6}$ alkylene group,
RfP is a phenyl group substituted with 1 to 5 fluorine atoms, and
n1 is an integer of 4 to 6.

[13] The compound according to any one of [1] to [12] above, wherein when the compound is formed into a film, a static contact angle of the film with water is 80° or more.
[14] A compound represented by Formula (1B) below:

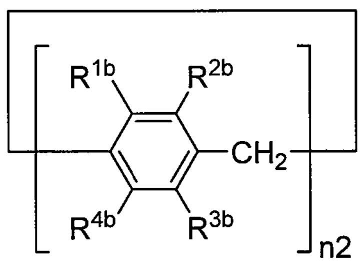

wherein

$R^{1b}$ is each independently at each occurrence a hydrogen atom or an organic group,
$R^{2b}$ is each independently at each occurrence a hydrogen atom or an organic group,
$R^{3b}$ is each independently at each occurrence a hydrogen atom or an organic group, and
$R^{4b}$ is each independently at each occurrence a hydrogen atom or an organic group,
provided that at least one of $R^{1b}$, $R^{2b}$, $R^{3b}$, and $R^{4b}$ is an organic group having a fluorine atom, and the organic group having a fluorine atom has an SP value of 8.0 or less, and n2 is an integer of 4 to 20.

[15] The compound according to [14] above, wherein

$R^{1b}$ and $R^{3b}$ are each independently an organic group, provided that at least one of $R^{1b}$ and $R^{3b}$ is an organic group having a fluorine atom and having an SP value of 8.0 or less, or
$R^{2b}$ and $R^{4b}$ are each independently an organic group, provided that at least one of $R^{2b}$ and $R^{4b}$ is an organic group having a fluorine atom and having an SP value of 8.0 or less.

[16] The compound according to [14] or [15] above, wherein $R^{1b}$ and $R^{3b}$ are each independently an organic group having a fluorine atom and having an SP value of 8.0 or less, or $R^{2b}$ and $R^{4b}$ are each independently an organic group having a fluorine atom and having an SP value of 8.0 or less.
[17] The compound according to any one of [14] to [16] above, wherein

$R^{2b}$ and $R^{4b}$ are each a hydrogen atom, or
$R^{1b}$ and $R^{3b}$ are each a hydrogen atom.

[18] The compound according to any one of [14] to [17] above, wherein n2 is an integer of 4 to 6.
[19] The compound according to any one of [14] to [18] above, wherein the SP value of the organic group having a fluorine atom is 4.0 to 7.5.

[20] The compound according to any one of [14] to [19] above, wherein the organic group having a fluorine atom has a $C_{1-10}$ fluoroalkyl group.
[21] The compound according to [30] above, wherein the $C_{1-10}$ fluoroalkyl group is a $C_{1-10}$ perfluoroalkyl group.
[22] The compound according to any one of [14] to [21] above, wherein

$R^{1b}$ and $R^{3b}$ are each -O-$R^{11b}$-(O-$R^{11b}$)$_{r2}$-$Rf^b$, and $R^{2b}$ and $R^{4b}$ are each a hydrogen atom, or
$R^{2b}$ and $R^{4b}$ are each -O-$R^{11b}$-(O-$R^{11b}$)$_{r2}$-$Rf^b$, and $R^{1b}$ and $R^{3b}$ are each a hydrogen atom,
$R^{11b}$ is each independently a $C_{1-10}$ alkylene group,
$Rf^b$ is a $C_{1-10}$ perfluoroalkyl group,
r2 is 0 or 1, and
n2 is an integer of 4 to 6.

[23] The compound according to any one of [14] to [22] above, wherein when the compound is formed into a film, a static contact angle of the film with water is 80° or more.
[24] A compound represented by Formula (1C) below:

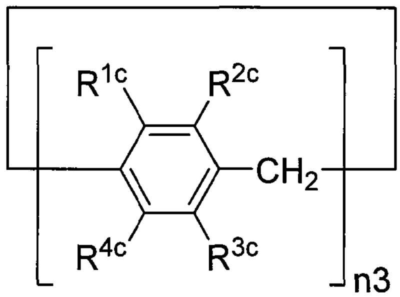

wherein

$R^{1c}$ is each independently at each occurrence a hydrogen atom or an organic group,
$R^{2c}$ is each independently at each occurrence a hydrogen atom or an organic group,
$R^{3c}$ is each independently at each occurrence a hydrogen atom or an organic group,
$R^{4c}$ is each independently at each occurrence a hydrogen atom or an organic group,
provided that at least one of $R^{1c}$, $R^{2c}$, $R^{3c}$, and $R^{4c}$ is an organic group having a long-chain alkyl group having 17 or more carbon atoms, and
n3 is an integer of 4 to 20.

[25] The compound according to [24] above, wherein

$R^{1c}$ and $R^{3c}$ are each independently an organic group,
provided that at least one of $R^{1c}$ and $R^{3c}$ is an organic group having a long-chain alkyl group having 17 or more carbon atoms, or
$R^{2c}$ and $R^{4c}$ are each independently an organic group,
provided that at least one of $R^{2c}$ and $R^{4c}$ is an organic group having a long-chain alkyl group having 17 or more carbon atoms.

[26] The compound according to [24] or [25] above, wherein $R^{1c}$ and $R^{3c}$ are each independently an organic group having a long-chain alkyl group having 17 or more carbon atoms, and $R^{2c}$ and $R^{4c}$ are each independently an organic group having a long-chain alkyl group having 17 or more carbon atoms.
[27] The compound according to any one of [24] to [26] above, wherein the number of carbon atoms in the long-chain alkyl group is 17 to 30.
[28] The compound according to any one of [24] to [27] above, wherein

$R^{1c}$ and $R^{3c}$ are each independently -O-$R^{12c}$, or
$R^{2c}$ and $R^{4c}$ are each independently -O-$R^{12c}$, and
$R^{12c}$ is a $C_{17-30}$ alkyl group.

[29] The compound according to any one of [24] to [28] above, wherein

$R^{2c}$ and $R^{4c}$ are each a hydrogen atom, or
$R^{1c}$ and $R^{3c}$ are each a hydrogen atom.

[30] The compound according to any one of [24] to [29] above, wherein n3 is an integer of 4 to 6.
[31] The compound according to any one of [24] to [30] above, wherein

$R^{1c}$ and $R^{3c}$ are each -O-$R^{12c}$, and $R^{2c}$ and $R^{4c}$ are each a hydrogen atom, or
$R^{2c}$ and $R^{4c}$ are each -O-$R^{12c}$, and $R^{1c}$ and $R^{3c}$ is each a hydrogen atom,
$R^{12c}$ is a $C_{17-30}$ alkyl group, and
n3 is an integer of 4 to 6.

[32] The compound according to any one of [24] to [31] above, wherein when the compound is formed into a film, a static contact angle of the film with water is 80° or more.
[33] A compound represented by Formula (1D) below:

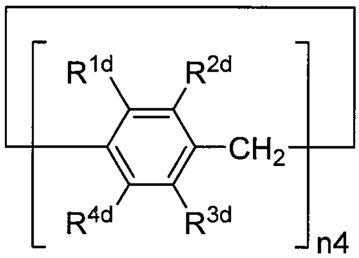

wherein

$R^{1d}$ is each independently at each occurrence a hydrogen atom or an organic group,
$R^{2d}$ is each independently at each occurrence a hydrogen atom or an organic group,
$R^{3d}$ is each independently at each occurrence a hydrogen atom or an organic group, and
$R^{4d}$ is each independently at each occurrence a hydrogen atom or an organic group,
provided that at least one of $R^{1d}$, $R^{2d}$, $R^{3d}$, and $R^{4d}$ is an organic group having an alkyl group, and the alkyl group has an SP value of 8.2 or more, and
n4 is an integer of 4 to 20.

[34] The compound according to [33] above, wherein

$R^{1d}$ and $R^{3d}$ are each independently an organic group,
provided that at least one of $R^{1d}$ and $R^{3d}$ is an organic group having an alkyl group having an SP value of 8.2 or more, or $R^{2d}$ and $R^{4d}$ are each independently an organic group,
provided that at least one of $R^{2d}$ and $R^{4d}$ is an organic group having an alkyl group having an SP value of 8.2 or more.

[35] The compound according to [33] or [34] above, wherein

$R^{1d}$ and $R^{3d}$ are each independently an organic group having an alkyl group having an SP value of 8.2 or more, or
$R^{2d}$ and $R^{4d}$ are each independently an organic group having an alkyl group having an SP value of 8.2 or more.

[36] The compound according to any one of [33] to [35] above, wherein

$R^{2d}$ and $R^{4d}$ are each a hydrogen atom, or
$R^{1d}$ and $R^{3d}$ are each a hydrogen atom.

[37] The compound according to any one of [33] to [36] above, wherein n4 is an integer of 4 to 6.
[38] The compound according to any one of [33] to [37] above, wherein the SP value of the alkyl group is 8.2 to 8.4.
[39] The compound according to any one of [33] to [38] above, wherein

$R^{1d}$ and $R^{3d}$ are each -O-$R^{12d}$, and $R^{2d}$ and $R^{4d}$ are each a hydrogen atom, or

$R^{2d}$ and $R^{4d}$ are each -O-$R^{12d}$, and $R^{1d}$ and $R^{3d}$ is each a hydrogen atom,
$R^{12d}$ is a $C_{17-30}$ alkyl group, and
n4 is an integer of 4 to 6.

[40] The compound according to any one of [33] to [39] above, wherein when the compound is formed into a film, a static contact angle of the film with water is 80° or more.
[41] A composition comprising at least one of the compound according to any one of [1] to [40] above.
[42] A formed article of the compound according to any one of [1] to [40] above.
[43] A formed article formed from the composition according to [41] above.
[44] The formed article according to [42] or [43] above, wherein the formed article is a film.
[45] A material comprising a substrate treated with the compound according to any one of [1] to [40] above or the composition according to [41] above.
[46] A liquid-repellent composition comprising a pillararene represented by Formula (1E) below and a hydrocarbon compound:

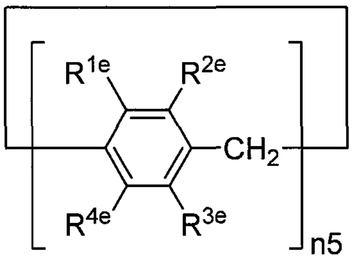

wherein

$R^{1e}$, $R^{2e}$, $R^{3e}$, and $R^{4e}$ are each independently a hydrogen atom or an organic group, and
n5 is 5 or 6.

[47] The liquid-repellent composition according to [46] above, wherein the organic group is an organic group having 1 to 30 carbon atoms.
[48] The liquid-repellent composition of [46] or [47] above, wherein the liquid-repellent composition further comprises a solvent.

Advantageous Effect of Invention

According to the present disclosure, a fluorinecontaining pillararene can be provided.

Description of Embodiments

As used herein, a "monovalent organic group" means a monovalent group containing carbon. The monovalent organic group is not limited, and can be a hydrocarbon group or a derivative thereof. A derivative of a hydrocarbon group means a group having one or more N, O, S, Si, amide, sulfonyl, siloxane, carbonyl, carbonyloxy, or the like at an end or in a molecular chain of the hydrocarbon group. When an "organic group" is simply shown, the organic group means a monovalent organic group.
As used herein, a "hydrocarbon group" means a group that contains carbon and hydrogen and that is obtained by removing one hydrogen atom from a hydrocarbon. The hydrocarbon group is not limited, and examples thereof include a $C_{1-20}$ hydrocarbon group optionally substituted with one or more substituents, such as an aliphatic hydrocarbon group or an aromatic hydrocarbon group. For example, the "aliphatic hydrocarbon group" may be linear, branched, or cyclic, and may be saturated or unsaturated. In addition, the hydrocarbon group may also include one or more ring structures.
As used herein, the substituent for the "hydrocarbon group" is not limited, and examples thereof include one or more groups selected from a halogen atom, a $C_{1-6}$ alkyl group, a $C_{2-6}$ alkenyl group, a $C_{2-6}$ alkynyl group, a $C_{3-10}$ cycloalkyl group, a $C_{3-10}$ unsaturated cycloalkyl group, a 5- to 10-membered heterocyclyl group, a 5- to 10-membered unsaturated heterocyclyl group, a $C_{6-10}$ aryl group, and a 5-to 10-membered heteroaryl group, optionally substituted with one or more halogen atoms.
In a first aspect, the present disclosure provides a compound represented by Formula (1A) below:

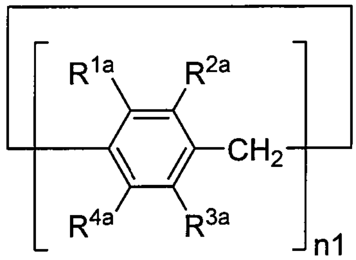

wherein

$R^{1a}$ is each independently at each occurrence a hydrogen atom or an organic group,
$R^{2a}$ is each independently at each occurrence a hydrogen atom or an organic group,
$R^{3a}$ is each independently at each occurrence a hydrogen atom or an organic group,
$R^{4a}$ is each independently at each occurrence a hydrogen atom or an organic group,
provided that at least one of $R^{1a}$, $R^{2a}$, $R^{3a}$, and $R^{4a}$ is an organic group having 2 or more carbon atoms and having a fluoroalkyl group, and
n1 is an integer of 4 to 20.

In Formula (1A) above, $R^{1a}$ is each independently at each occurrence a hydrogen atom or an organic group, $R^{2a}$ is each independently at each occurrence a hydrogen atom or an organic group, $R^{3a}$ is each independently at each occurrence a hydrogen atom or an organic group, $R^{4a}$ is each independently at each occurrence a hydrogen atom or an organic group, and at least one of $R^{1a}$, $R^{2a}$, $R^{3a}$, and $R^{4a}$ is an organic group having 2 or more carbon atoms and having a fluoroalkyl group or a fluorophenyl group. In one embodiment, at least one of $R^{1a}$, $R^{2a}$, $R^{3a}$, and $R^{4a}$ is an organic group having 2 or more carbon atoms and having a fluoroalkyl group.

The compound represented by Formula (1A) can have high miscibility, heat resistance, and water and oil repellency by having an organic group having 2 or more carbon atoms and having a fluoroalkyl group or a fluorophenyl group.

Examples of the organic group include a group including an alkyl group, an alkyloxy group, an alkyl ether group, a tosyl group, a triflate group, a phenyl group, or the like, optionally substituted with a substituent.

The substituent is not limited, and examples thereof include one or more groups selected from a halogen atom, a $C_{1-6}$ alkyl group, a $C_{2-6}$ alkenyl group, a $C_{2-6}$ alkynyl group, a $C_{3-10}$ cycloalkyl group, a $C_{3-10}$ unsaturated cycloalkyl group, a 5- to 10-membered heterocyclyl group, a 5- to 10-membered unsaturated heterocyclyl group, a $C_{6-10}$ aryl group, and a 5- to 10-membered heteroaryl group, optionally substituted with one or more halogen atoms, and a reactive functional group.

The reactive functional group is not limited, and examples thereof include a hydroxyl group, an amino group, a carboxyl group, a thiol group, an isocyanate group, a vinyl group, an acetylene group, a nitrile group, and an epoxy group.

The alkyl group is preferably a $C_{1-30}$ alkyl group. For example, the alkyl group may be linear or branched. In one embodiment, the $C_{1-30}$ alkyl group can be a $C_{1-20}$ alkyl group, preferably a $C_{1-10}$ alkyl group, and more preferably a $C_{1-30}$ alkyl group. In another embodiment, the $C_{1-30}$ alkyl group can be a $C_{17-30}$ alkyl group, preferably a $C_{18-30}$ alkyl group, and more preferably a $C_{26-30}$ alkyl group.

The alkyloxy group is typically a group represented by $-O-R^{31}$ wherein $R^{31}$ is an alkyl group.

The alkyl group of $R^{31}$ is optionally substituted with any of the substituents described above, and preferably optionally substituted with a fluorine atom. The alkyl group is preferably a $C_{1-30}$ alkyl group optionally substituted with a fluorine atom. For example, the alkyl group may be linear or branched. In one embodiment, the $C_{1-30}$ alkyl group can be a $C_{1-20}$ alkyl group, preferably a $C_{1-10}$ alkyl group, and more preferably a $C_{1-30}$ alkyl group.

$R^{31}$ is a group represented by preferably $-R^{32}-R^{33}$ wherein $R^{32}$ is an unsubstituted $C_{1-30}$ alkylene group, preferably $C_{1-20}$ alkylene group, and more preferably $C_{1-10}$ alkylene group, and $R^{33}$ is a $C_{1-10}$ perfluoroalkyl group, and preferably a $C_{1-6}$ perfluoroalkyl group.

The alkyl ether group is a compound having one or more ethereal oxygen atoms in a molecular chain of the alkyl group. The alkyl ether group is typically a group represented by $-R^{36}-R^{37}-(O-R^{38})_m-R^{39}$. In the above formula, $R^{36}$ is a single bond or an oxygen atom. $R^{37}$ is a single bond or a $C_{1-10}$ alkylene group. $R^{38}$ is a $C_{1-10}$ alkylene group. $R^{39}$ is a $C_{1-10}$ alkyl group optionally substituted with a fluorine atom.

In one embodiment, the organic group can be an alkyl group, an alkyloxy group, or an alkyl ether group, having an end substituted with a reactive functional group. The group is optionally further substituted with another substituent.

In one embodiment, $R^{1a}$ and $R^{3a}$ are each independently an organic group, and at least one of $R^{1a}$ and $R^{3a}$ is an organic group having 2 or more carbon atoms and having a fluoroalkyl group or a fluorophenyl group, preferably a

fluoroalkyl group, or $R^{2a}$ and $R^{4a}$ are each independently an organic group, and at least one of $R^{2a}$ and $R^{4a}$ is an organic group having 2 or more carbon atoms and having a fluoroalkyl group or a fluorophenyl group, preferably a fluoroalkyl group.

In one embodiment, at least two of $R^{1a}$, $R^{2a}$, $R^{3a}$, and $R^{4a}$ are each an organic group having 2 or more carbon atoms and having a fluoroalkyl group or a fluorophenyl group. In one embodiment, at least two of $R^{1a}$, $R^{2a}$, $R^{3a}$, and $R^{4a}$ are each an organic group having 2 or more carbon atoms and having a fluoroalkyl group.

In one embodiment, two of $R^{1a}$, $R^{2a}$, $R^{3a}$, and $R^{4a}$ are each an organic group having 2 or more carbon atoms and having a fluoroalkyl group or a fluorophenyl group. In one embodiment, two of $R^{1a}$, $R^{2a}$, $R^{3a}$, and $R^{4a}$ are each an organic group having 2 or more carbon atoms and having a fluoroalkyl group.

In one embodiment, $R^{1a}$ and $R^{3a}$ are each independently an organic group having 2 or more carbon atoms and having a fluoroalkyl group or a fluorophenyl group, preferably a fluoroalkyl group, or $R^{2a}$ and $R^{4a}$ are each independently an organic group having 2 or more carbon atoms and having a fluoroalkyl group or a fluorophenyl group, preferably a fluoroalkyl group.

In one embodiment, $R^{2a}$ and $R^{4a}$ are each a hydrogen atom, or $R^{1a}$ and $R^{3a}$ are each a hydrogen atom.

In a preferable embodiment, $R^{1a}$ and $R^{3a}$ are each independently an organic group having 2 or more carbon atoms and having a fluoroalkyl group or a fluorophenyl group, preferably a fluoroalkyl group, and $R^{2a}$ and $R^{4a}$ are each a hydrogen atom, or $R^{2a}$ and $R^{4a}$ are each independently an organic group having 2 or more carbon atoms and having a fluoroalkyl group or a fluorophenyl group, preferably a fluoroalkyl group, and $R^{1a}$ and $R^{3a}$ are each a hydrogen atom.

In a more preferable embodiment, $R^{1a}$ and $R^{3a}$ are each an organic group having 2 or more carbon atoms and having a fluoroalkyl group or a fluorophenyl group, preferably a fluoroalkyl group, and $R^{2a}$ and $R^{4a}$ are each a hydrogen atom, or $R^{2a}$ and $R^{4a}$ are each an organic group having 2 or more carbon atoms and having a fluoroalkyl group or a fluorophenyl group, preferably a fluoroalkyl group, and $R^{1a}$ and $R^{3a}$ are each a hydrogen atom.

The fluoroalkyl group is preferably a $C_{1-10}$ fluoroalkyl group, and more preferably a $C_{1-6}$ fluoroalkyl group. For example, the fluoroalkyl group may be linear or branched, and is preferably linear. The fluoroalkyl group is preferably a perfluoroalkyl group.

For example, the fluorophenyl group may be a phenyl group substituted with 1 to 5 fluorine atoms, and is preferably a phenyl group substituted with 5 fluorine atoms.

In one embodiment, the organic group having 2 or more carbon atoms having a fluoroalkyl group is a group represented by the following formula:

$$-(O_{p1}-R^{11a}{}_{q1})-Rf^a$$

wherein

$R^{11a}$ is each independently at each occurrence a $C_{1-10}$ alkylene group or -CONH-,
$Rf^a$ is a $C_{1-10}$ fluoroalkyl group,
p1 is an integer of 0 to 2,
q1 is an integer of 0 to 3, and
in $(O_{p1}-R^{11a}{}_{q}1)$, occurrence order of O and $R^{11a}$ is not limited.

The $C_{1-10}$ alkylene group in $R^{11a}$ is preferably a $C_{1-6}$ alkylene group, and more preferably a $C_{2-6}$ alkylene group. For example, the alkylene group may be linear or branched, and is preferably linear.

In one embodiment, $R^{11a}$ is each independently at each occurrence a $C_{1-10}$ alkylene group.

In another embodiment, $R^{11a}$ is each independently at each occurrence -CONH-.

The $C_{1-10}$ fluoroalkyl group in $Rf^a$ is preferably a $C_{1-6}$ fluoroalkyl group. For example, the fluoroalkyl group may be linear or branched, and is preferably linear. The fluoroalkyl group is preferably a perfluoroalkyl group.

p1 is an integer of 0 to 2, and is preferably 1 or 2. In one embodiment, p1 is 1. In another embodiment, p1 is 2.

q1 is an integer of 0 to 3, and preferably an integer of 1 to 3, and is more preferably 1 or 2. In one embodiment, q1 is 1. In another embodiment, q1 is 2.

In a preferable embodiment, the organic group having 2 or more carbon atoms and having a fluoroalkyl group is a group represented by

$$-O-R^{11a}-(O-R^{11a})_{r1}-Rf^a$$

wherein

$R^{11a}$ is each independently a $C_{1-10}$ alkylene group or -CONH-,
$Rf^a$ is a $C_{1-10}$ fluoroalkyl group, and
r1 is 0 or 1.

In a more preferable embodiment, the organic group having 2 or more carbon atoms and having a fluoroalkyl group is a group represented by

$$-O-R^{11a}-(O-R^{11a})_{r1}-Rf^{a}$$

wherein

$R^{11a}$ is each independently a $C_{1-10}$ alkylene group or -CONH-,
$Rf^a$ is a $C_{1-10}$ perfluoroalkyl group, and
r1 is 0 or 1.

In one aspect, the organic group having 2 or more carbon atoms and having a fluorophenyl group is a group represented by the following formula:

$$-(O_{p3}-R^{11p}{}_{q3})-Rf^{p}$$

wherein

$R^{11p}$ is each independently at each occurrence a $C_{1-10}$ alkylene group, preferably a $C_{1-6}$ alkylene group, and more preferably a $C_{1-3}$ alkylene group,
RfP is a phenyl group substituted with 1 to 5, preferably 5, fluorine atoms,
p3 is an integer of 0 to 2,
q3 is an integer of 0 to 3, and
in $(O_{P3}-R^{11p}{}_{q3})$, occurrence order of O and $R^{11p}$ is not limited.

The $C_{1-10}$ alkylene group in $R^{11p}$ is preferably a $C_{1-6}$ alkylene group, and more preferably a $C_{1-6}$ alkylene group. For example, the alkylene group may be linear or branched, and is preferably linear.
p3 is an integer of 0 to 2, and is preferably 1 or 2. In one embodiment, p1 is 1. In another embodiment, p1 is 2.
q3 is an integer of 0 to 3, and preferably an integer of 1 to 3, and is more preferably 1 or 2. In one embodiment, q1 is 1. In another embodiment, q1 is 2.
In a preferable embodiment, the organic group having 2 or more carbon atoms and having a fluorophenyl group is a group represented by the following formula:

$$-O-R^{11p}-Rf^{p}$$

wherein

$R^{11p}$ is each independently at each occurrence a $C_{1-10}$ alkylene group, preferably a $C_{1-6}$ alkylene group, and more preferably a $C_{1-3}$ alkylene group, and
RfP is a phenyl group substituted with 1 to 5, preferably 5, fluorine atoms.
n1 is an integer of 4 to 20, and preferably an integer of 4 to 6, and is more preferably 5 or 6.

In a particularly preferable embodiment,

$R^{1a}$ and $R^{3a}$ are each $-O-R^{11a}-(O-R^{11a})_{r1}-Rf^{a}$, and $R^{2a}$ and $R^{4a}$ are each a hydrogen atom, or
$R^{2a}$ and $R^{4a}$ are each $-O-R^{11a}-(O-R^{11a})_{r1}-Rf^{a}$, and $R^{1a}$ and $R^{3a}$ are each a hydrogen atom,
$R^{11a}$ is each independently a $C_{1-10}$ alkylene group, and preferably a $C_{1-6}$ alkylene group,
$Rf^a$ is a $C_{1-10}$ perfluoroalkyl group, and preferably a $C_{1-6}$ perfluoroalkyl group,
r1 is 0 or 1, and
n1 is an integer of 4 to 6, and is preferably 5 or 6.

In another particularly preferable embodiment,

$R^{1a}$ and $R^{3a}$ are each $-O-R^{11p}-Rf^{p}$, and $R^{2a}$ and $R^{4a}$ are each a hydrogen atom, or
$R^{2a}$ and $R^{4a}$ are $-O-R^{11p}-Rf^{p}$, and $R^{1a}$ and $R^{3a}$ are each a hydrogen atom,
$R^{13p}$ is each independently a $C_{1-6}$ alkylene group, and preferably a $C_{1-3}$ alkylene group,
RfP is a phenyl group substituted with 1 to 5, preferably 5, fluorine atoms, and
n1 is an integer of 4 to 6, and is preferably 5 or 6.

The molecular weight of the compound represented by Formula (1A) above can be preferably 500 to 10,000, and more preferably 1,000 to 5,000.

When the compound represented by Formula (1A) above is formed into a film, the static contact angle of the resulting film with water is 80° or more, and preferably 90° or more.

The compound represented by Formula (1A) above can be obtained by converting a substituent of a pillar[n]arene to an organic group having 2 or more carbon atoms and having a fluoroalkyl group. Typically, the compound represented by Formula (1A) above can be obtained by introducing a hydroxyl group at a predetermined position of a pillar[n]arene and reacting the hydroxyl group with a tosylate, a halide, or the like of an organic group having 2 or more carbon atoms and having a fluoroalkyl group.

In a second aspect, the present disclosure provides a compound represented by Formula (1B) below:

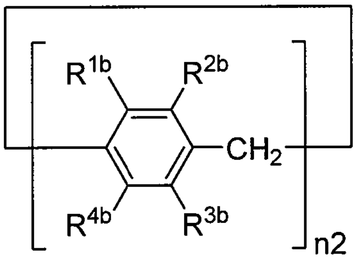

wherein

- $R^{1b}$ is each independently at each occurrence a hydrogen atom or an organic group,
- $R^{2b}$ is each independently at each occurrence a hydrogen atom or an organic group,
- $R^{3b}$ is each independently at each occurrence a hydrogen atom or an organic group, and
- $R^{4b}$ is each independently at each occurrence a hydrogen atom or an organic group,
- provided that at least one of $R^{1b}$, $R^{2b}$, $R^{3b}$, and $R^{4b}$ is an organic group having a fluorine atom, and the organic group having a fluorine atom has an SP value of 8.0 or less, and
- n2 is an integer of 4 to 20.

As used herein, the SP value is a solubility parameter and serves as a measure of solubility. The SP value is a value unique to a substance; and a larger numerical value thereof shows higher polarity, and a smaller numerical value thereof shows lower polarity. The SP value is the value $\delta(cal/cm^3)^{1/2}$ calculated from the following expression after determining the evaporation energy ($\Delta ei$) and the molar volume ($\Delta vi$) by using the Fedors method (R.F. Fedors. Polyme. Eng. Sic., 14, 147(1974)).

$$\delta = (\sum\Delta ei/\sum\Delta vi)^{1/2}$$

In Formula (1B) above, $R^{1b}$ is each independently at each occurrence a hydrogen atom or an organic group, $R^{2b}$ is each independently at each occurrence a hydrogen atom or an organic group, $R^{3b}$ is each independently at each occurrence a hydrogen atom or an organic group, $R^{4b}$ is each independently at each occurrence a hydrogen atom or an organic group, at least one of $R^{1b}$, $R^{2b}$, $R^{3b}$, and $R^{4b}$ is an organic group having a fluorine atom, and the organic group having a fluorine atom has an SP value of 8.0 or less.

The compound represented by Formula (1B) can have high miscibility, heat resistance, and water and oil repellency by having an organic group having a fluorine atom and having an SP value of 8.0 or less.

The organic group is defined as described for the organic group in Formula (1A) above.

In one embodiment, $R^{1b}$ and $R^{3b}$ are each independently an organic group, and at least one of $R^{1b}$ and $R^{3b}$ is an organic group having a fluorine atom and having an SP value of 8.0 or less, or $R^{2b}$ and $R^{4b}$ are each independently an organic group, and at least one of $R^{2b}$ and $R^{4b}$ is an organic group having a fluorine atom and having an SP value of 8.0 or less.

In one embodiment, at least two of $R^{1b}$, $R^{2b}$, $R^{3b}$, and $R^{4b}$ are each an organic group having a fluorine atom and having an SP value of 8.0 or less.

In one embodiment, two of $R^{1b}$, $R^{2b}$, $R^{3b}$, and $R^{4b}$ are each an organic group having a fluorine atom and having an SP value of 8.0 or less.

$R^{1b}$ and $R^{3b}$ are each independently an organic group having a fluorine atom and having an SP value of 8.0 or less, or $R^{2b}$ and $R^{4b}$ are each independently an organic group having a fluorine atom and having an SP value of 8.0

or less.

In one embodiment, $R^{2b}$ and $R^{4b}$ are each a hydrogen atom, or $R^{1b}$ and $R^{3b}$ are each a hydrogen atom.

In a preferable embodiment, $R^{1b}$ and $R^{3b}$ are each independently an organic group having a fluorine atom and having an SP value of 8.0 or less, and $R^{2b}$ and $R^{4b}$ are each a hydrogen atom, or $R^{2b}$ and $R^{4b}$ are each independently an organic group having a fluorine atom and having an SP value of 8.0 or less, and $R^{2b}$ and $R^{4b}$ are each a hydrogen atom.

In a more preferable embodiment, $R^{1b}$ and $R^{3b}$ are each an organic group having a fluorine atom and having an SP value of 8.0 or less, and $R^{2b}$ and $R^{4b}$ are each a hydrogen atom, or $R^{2b}$ and $R^{4b}$ are each an organic group having a fluorine atom and having an SP value of 8.0 or less, and $R^{1b}$ and $R^{3b}$ are each a hydrogen atom.

The SP value of the organic group having a fluorine atom is preferably 4.0 to 7.5, and more preferably 6.0 to 7.5.

In a preferable embodiment, the organic group having a fluorine atom has a $C_{1-10}$ fluoroalkyl group. The fluoroalkyl group is preferably a $C_{1-6}$ fluoroalkyl group. For example, the fluoroalkyl group may be linear or branched, and is preferably linear. The fluoroalkyl group is preferably a perfluoroalkyl group.

In another preferable embodiment, the organic group having a fluorine atom has a fluorophenyl group. The fluorophenyl group is a phenyl group substituted with 1 to 5, preferably 5, fluorine atoms.

In one embodiment, the organic group having a fluorine atom is a group represented by the following formula:

$$-(O_{p2}-R^{11b}{}_{q2})-Rf^{b}$$

wherein

$R^{11b}$ is each independently at each occurrence a $C_{1-10}$ alkylene group,
$Rf^{b}$ is a $C_{1-10}$ fluoroalkyl group,
p2 is an integer of 0 to 2,
q2 is an integer of 0 to 3, and
in $(O_{p2}-R^{11b}{}_{q2})$, occurrence order of O and $R^{11b}$ is not limited.

In one embodiment, the organic group having 2 or more carbon atoms having a fluorophenyl group is a group represented by the following formula:

$$-(O_{p1}-R^{11a}{}_{q1})-Rf^{p}$$

wherein

$R^{11a}$ is each independently at each occurrence a $C_{1-10}$ alkylene group,
RfP is a phenyl group substituted with 1 to 5, preferably 5, fluorine atoms,
p1 is an integer of 0 to 2,
q1 is an integer of 0 to 3, and
in $(O_{p1}-R^{11a}{}_{q1})$, occurrence order of O and $R^{11a}$ is not limited.

The $C_{1-10}$ alkylene group in $R^{11b}$ is preferably a $C_{1-6}$ alkylene group, and more preferably a $C_{2-6}$ alkylene group. For example, the alkylene group may be linear or branched, and is preferably linear.

The $C_{1-10}$ fluoroalkyl group in $Rf^{b}$ is preferably a $C_{1-6}$ fluoroalkyl group. For example, the fluoroalkyl group may be linear or branched, and is preferably linear. The fluoroalkyl group is preferably a perfluoroalkyl group.

p2 is an integer of 0 to 2, and is preferably 1 or 2. In one embodiment, p2 is 1. In another embodiment, p2 is 2.

q2 is an integer of 0 to 3, and preferably an integer of 1 to 3, and is more preferably 1 or 2. In one embodiment, q2 is 1. In another embodiment, q2 is 2.

In a preferable embodiment, the organic group having a fluorine atom is a group represented by

$$-O-R^{11b}-(O-R^{11b})_{r2}-Rf^{b}$$

wherein

$R^{11b}$ is each independently a $C_{1-10}$ alkylene group,
$Rf^{b}$ is a $C_{1-10}$ fluoroalkyl group, and
r2 is 0 or 1.

In a more preferable embodiment, the organic group having a fluorine atom is a group represented by

$-O-R^{11b}-(O-R^{11b})_{r2}-Rf^b$

wherein

$R^{11b}$ is each independently a $C_{1-10}$ alkylene group,
$Rf^b$ is a $C_{1-10}$ fluoroalkyl group, and
r2 is 0 or 1.

n2 is an integer of 4 to 20, and preferably an integer of 4 to 6, and is more preferably 5 or 6.
In a particularly preferable embodiment,

$R^{1b}$ and $R^{3b}$ are each $-O-R^{11b}-(O-R^{11b})_{r2}-Rf^b$, and $R^{2b}$ and $R^{4b}$ are each a hydrogen atom, or
$R^{2b}$ and $R^{4b}$ are each $-O-R^{11b}-(O-R^{11b})_{r2}-Rf^b$, and $R^{1b}$ and $R^{3b}$ are each a hydrogen atom,
$R^{11b}$ is each independently a $C_{1-10}$ alkylene group, and preferably a $C_{1-6}$ alkylene group,
$Rf^b$ is a $C_{1-10}$ perfluoroalkyl group, and preferably a $C_{1-6}$ perfluoroalkyl group,
r2 is 0 or 1, and
n2 is an integer of 4 to 6, and is preferably 5 or 6.

The molecular weight of the compound represented by Formula (1B) above can be preferably 500 to 10,000, and more preferably 1,000 to 5,000.
When the compound represented by Formula (1B) above is formed into a film, the static contact angle of the resulting film with water is 80° or more, and preferably 90° or more.
The compound represented by Formula (1B) above can be produced by the same method as for the compound represented by Formula (1A) above. For example, the compound represented by Formula (1B) above can be obtained by introducing a hydroxyl group at a predetermined position of a pillar[n]arene and reacting the hydroxyl group with a tosylate, a halide, or the like of an organic group having a fluorine atom.
In a third aspect, the present disclosure provides a compound represented by Formula (1C) below:

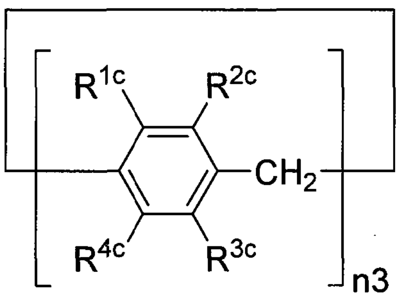

wherein

$R^{1c}$ is each independently at each occurrence a hydrogen atom or an organic group,
$R^{2c}$ is each independently at each occurrence a hydrogen atom or an organic group,
$R^{3c}$ is each independently at each occurrence a hydrogen atom or an organic group,
$R^{4c}$ is each independently at each occurrence a hydrogen atom or an organic group,
provided that at least one of $R^{1c}$, $R^{2c}$, $R^{3c}$, and $R^{4c}$ is an organic group having a long-chain alkyl group having 17 or more carbon atoms, and
n3 is an integer of 4 to 20.

In Formula (1C) above, $R^{1c}$ is each independently at each occurrence a hydrogen atom or an organic group, $R^{2c}$ is each independently at each occurrence a hydrogen atom or an organic group, $R^{3c}$ is each independently at each occurrence a hydrogen atom or an organic group, $R^{4c}$ is each independently at each occurrence a hydrogen atom or an organic group, and at least one of $R^{1c}$, $R^{2c}$, $R^{3c}$, and $R^{4c}$ is an organic group having a long-chain alkyl group having 17 or more carbon atoms.
The compound represented by Formula (1C) can have high miscibility, heat resistance, and water repellency by having an organic group having a long-chain alkyl group having 17 or more carbon atoms.
The organic group is defined as described for the organic group in Formula (1A) above.
The number of carbon atoms in the long-chain alkyl group is preferably 17 to 30, more preferably 18 to 30, and

further preferably 26 to 30.

The organic group having a long-chain alkyl group is preferably a group represented by -O-$R^{12c}$ wherein $R^{12c}$ is a long-chain alkyl group having 17 or more carbon atoms, preferably a $C_{17-30}$ alkyl group, and preferably a $C_{18-30}$ alkyl group or a $C_{26-30}$ alkyl group.

For example, the long-chain alkyl group may be linear or branched, and is preferably linear.

In one embodiment, $R^{1c}$ and $R^{3c}$ are each independently an organic group, and at least one of $R^{1c}$ and $R^{3c}$ is an organic group having a long-chain alkyl group having 17 or more carbon atoms, or $R^{2c}$ and $R^{4c}$ are each independently an organic group, and at least one of $R^{2c}$ and $R^{4c}$ is an organic group having a long-chain alkyl group having 17 or more carbon atoms.

In one embodiment, at least two of $R^{1c}$, $R^{2c}$, $R^{3c}$, and $R^{4c}$ are each an organic group having a long-chain alkyl group having 17 or more carbon atoms.

In one embodiment, two of $R^{1c}$, $R^{2c}$, $R^{3c}$, and $R^{4c}$ are each an organic group having a long-chain alkyl group having 17 or more carbon atoms.

In one embodiment, $R^{1c}$ and $R^{3c}$ are each independently an organic group having a long-chain alkyl group having 17 or more carbon atoms, and preferably -O-$R^{12c}$, or $R^{2c}$ and $R^{4c}$ are each independently an organic group having a long-chain alkyl group having 17 or more carbon atoms, and preferably -O-$R^{12c}$.

In one embodiment, $R^{2c}$ and $R^{4c}$ are each a hydrogen atom, or $R^{1c}$ and $R^{3c}$ are each a hydrogen atom.

In a preferable embodiment, $R^{1c}$ and $R^{3c}$ are each independently an organic group having a long-chain alkyl group having 17 or more carbon atoms, and $R^{2c}$ and $R^{4c}$ are each a hydrogen atom, or $R^{2c}$ and $R^{4c}$ are each independently an organic group having a long-chain alkyl group having 17 or more carbon atoms, and $R^{1c}$ and $R^{3c}$ are each a hydrogen atom.

In a more preferable embodiment, $R^{1c}$ and $R^{3c}$ are each an organic group having a long-chain alkyl group having 17 or more carbon atoms, and $R^{2c}$ and $R^{4c}$ are each a hydrogen atom, or $R^{2c}$ and $R^{4c}$ are each an organic group having a long-chain alkyl group having 17 or more carbon atoms, and $R^{1c}$ and $R^{3c}$ are each a hydrogen atom.

n3 is an integer of 4 to 20, and preferably an integer of 4 to 6, and is more preferably 5 or 6.

In a particularly preferable embodiment,

$R^{1c}$ and $R^{3c}$ are each -O-$R^{12c}$, and $R^{2c}$ and $R^{4c}$ are each a hydrogen atom, or
$R^{2c}$ and $R^{4c}$ are each -O-$R^{12c}$, and $R^{1c}$ and $R^{3c}$ is each a hydrogen atom,
$R^{12c}$ is a $C_{17-30}$ alkyl group, and preferably a $C_{18-30}$ alkyl group or a $C_{26-30}$ alkyl group, and
n3 is an integer of 4 to 6, and is preferably 5 or 6.

The molecular weight of the compound represented by Formula (1C) above can be preferably 500 to 10,000, and more preferably 1,000 to 5,000.

When the compound represented by Formula (1C) above is formed into a film, the static contact angle of the resulting film with water is 80° or more, and preferably 90° or more.

The compound represented by Formula (1C) above can be produced by the same method as for the compound represented by Formula (1A) above. For example, the compound represented by Formula (1C) above can be obtained by introducing a hydroxyl group at a predetermined position of a pillar[n]arene and reacting the hydroxyl group with a tosylate, a halide, or the like of an organic group having a long-chain alkyl group having 17 or more carbon atoms.

In a fourth aspect, the present disclosure provides a compound represented by Formula (1D) below:

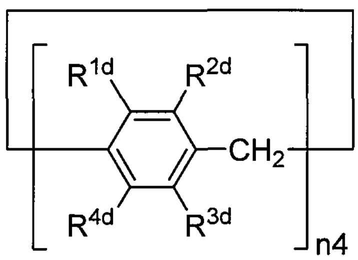

wherein

$R^{1d}$ is each independently at each occurrence a hydrogen atom or an organic group,
$R^{2d}$ is each independently at each occurrence a hydrogen atom or an organic group,
$R^{3d}$ is each independently at each occurrence a hydrogen atom or an organic group, and
$R^{4d}$ is each independently at each occurrence a hydrogen atom or an organic group,

provided that at least one of $R^{1d}$, $R^{2d}$, $R^{3d}$, and $R^{4d}$ is an organic group having an alkyl group, and the alkyl group has an SP value of 8.2 or more, and
n4 is an integer of 4 to 20.

In Formula (1D) above, $R^{1d}$ is each independently at each occurrence a hydrogen atom or an organic group, $R^{2d}$ is each independently at each occurrence a hydrogen atom or an organic group, $R^{3d}$ is each independently at each occurrence a hydrogen atom or an organic group, $R^{4d}$ is each independently at each occurrence a hydrogen atom or an organic group, at least one of $R^{1d}$, $R^{2d}$, $R^{3d}$, and $R^{4d}$ is an organic group having an alkyl group, and the alkyl group has an SP value of 8.2 or more.

The compound represented by Formula (1D) can have high miscibility, heat resistance, and water repellency by having an organic group having an alkyl group having an SP value of 8.2 or more.

The organic group is defined as described for the organic group in Formula (1A) above.

In one embodiment, $R^{1d}$ and $R^{3d}$ are each independently an organic group, and at least one of $R^{1d}$ and $R^{3d}$ is an organic group having an alkyl group having an SP value of 8.2 or more, or $R^{2d}$ and $R^{4d}$ are each independently an organic group, and at least one of $R^{2d}$ and $R^{4d}$ is an organic group having an alkyl group having an SP value of 8.2 or more.

In one embodiment, at least two of $R^{1d}$, $R^{2b}$, $R^{3d}$, and $R^{4d}$ are each an organic group having an alkyl group having an SP value of 8.2 or more.

In one embodiment, two of $R^{1d}$, $R^{2d}$, $R^{3d}$, and $R^{4d}$ are each an organic group having an alkyl group having an SP value of 8.2 or more.

In one embodiment, $R^{1d}$ and $R^{3d}$ are each independently an organic group having an alkyl group having an SP value of 8.2 or more, or $R^{2d}$ and $R^{4d}$ are each independently an organic group having an alkyl group having an SP value of 8.2 or more.

In one embodiment, $R^{2d}$ and $R^{4d}$ are each a hydrogen atom, or $R^{1d}$ and $R^{3d}$ are each a hydrogen atom.

In a preferable embodiment, $R^{1d}$ and $R^{3d}$ are each independently an organic group having an alkyl group having an SP value of 8.2 or more, and $R^{2d}$ and $R^{4d}$ are each a hydrogen atom, or $R^{2d}$ and $R^{4d}$ are each independently an organic group having an alkyl group having an SP value of 8.2 or more, and $R^{1d}$ and $R^{3d}$ are each a hydrogen atom.

In a more preferable embodiment, $R^{1d}$ and $R^{3d}$ are each an organic group having an alkyl group having an SP value of 8.2 or more, and $R^{2d}$ and $R^{4d}$ are each a hydrogen atom, or $R^{2d}$ and $R^{4d}$ are each an organic group having an alkyl group having an SP value of 8.2 or more, and $R^{1d}$ and $R^{3d}$ are each a hydrogen atom.

The SP value of the alkyl group is preferably 8.2 to 8.4.

In a preferable embodiment, the organic group having an alkyl group is preferably a group represented by -O-$R^{12d}$ wherein $R^{12d}$ is a long-chain alkyl group having 17 or more carbon atoms, preferably a $C_{17-30}$ alkyl group, and preferably a $C_{18-30}$ alkyl group or a $C_{26-30}$ alkyl group.

For example, the long-chain alkyl group may be linear or branched, and is preferably linear.

n4 is an integer of 4 to 20, and preferably an integer of 4 to 6, and is more preferably 5 or 6.

In a particularly preferable embodiment,

the compound according to any one of claims 30 to 35, wherein $R^{1d}$ and $R^{3d}$ are each -O-$R^{12d}$, and $R^{2d}$ and $R^{4d}$ are each a hydrogen atom, or
$R^{2d}$ and $R^{4d}$ are each -O-$R^{12d}$, and $R^{1d}$ and $R^{3d}$ is each a hydrogen atom,
$R^{12d}$ is a $C_{17-30}$ alkyl group, and preferably a $C_{18-30}$ alkyl group or a $C_{26-30}$ alkyl group, and
n4 is an integer of 4 to 6, and is preferably 5 or 6.

The molecular weight of the compound represented by Formula (1D) above can be preferably 500 to 10,000, and more preferably 1,000 to 5,000.

When the compound represented by Formula (1D) above is formed into a film, the static contact angle of the resulting film with water is 80° or more, and preferably 90° or more.

The compound represented by Formula (1D) above can be produced by the same method as for the compound represented by Formula (1A) above. For example, the compound represented by Formula (1D) above can be obtained by introducing a hydroxyl group at a predetermined position of a pillar[n]arene and reacting the hydroxyl group with a tosylate, a halide, or the like of an organic group having an alkyl group.

The present disclosure provides a composition including a compound represented by Formula (IA), (1B), (1C), or (1D) above.

The composition of the present disclosure may include the compounds of the present disclosure singly or may include two or more thereof in combination.

For example, the composition of the present disclosure may be a liquid or a solid.

In the composition of the present disclosure, each component may be included as a so-called inclusion, which is what is obtained by including a further component in the pillararene of the present disclosure, or for example, may be

a so-called surface adhered substance, which is what is obtained by adhering a further component to the pillararene of the present disclosure.

The composition of the present disclosure may include a solvent in addition to the compound of the present disclosure.

Examples of the solvent include water; an aliphatic hydrocarbon such as hexane, cyclohexane, heptane, octane, nonane, decane, undecane, dodecane, or a mineral spirit; an aromatic hydrocarbon such as benzene, toluene, xylene, naphthalene, or solvent naphtha; an ester such as methyl acetate, ethyl acetate, propyl acetate, n-butyl acetate, isopropyl acetate, isobutyl acetate, cellosolve acetate, propylene glycol methyl ether acetate, carbitol acetate, diethyl oxalate, ethyl pyruvate, ethyl-2-hydroxybutyrate, ethyl acetoacetate, amyl acetate, methyl lactate, ethyl lactate, methyl 3-methoxypropionate, ethyl 3-methoxypropionate, methyl 2-hydroxyisobutyrate, or ethyl 2-hydroxyisobutyrate; a ketone such as acetone, methyl ethyl ketone, methyl isobutyl ketone, 2-hexanone, cyclohexanone, methylamino ketone, or 2-heptanone; a glycol ether such as ethyl cellosolve, methyl cellosolve, methyl cellosolve acetate, ethyl cellosolve acetate, propylene glycol monomethyl ether, propylene glycol monoethyl ether, propylene glycol monobutyl ether, propylene glycol monomethyl ether acetate, propylene glycol monoethyl ether acetate, propylene glycol monobutyl ether acetate, dipropylene glycol dimethyl ether, or ethylene glycol monoalkyl ether; an alcohol such as methanol, ethanol, isopropanol, n-butanol, isobutanol, tert-butanol, sec-butanol, 3-pentanol, octyl alcohol, 3-methyl-3-methoxybutanol, or tert-amyl alcohol; a glycol such as ethylene glycol or propylene glycol; a cyclic ether such as tetrahydrofuran, tetrahydropyran, or dioxane; an amide such as N,N-dimethylformamide or N,N-dimethylacetamide; an ether alcohol such as methyl cellosolve, cellosolve, isopropyl cellosolve, butyl cellosolve, or diethylene glycol monomethyl ether; diethylene glycol monoethyl ether acetate; and a fluorinecontaining solvent such as 1,1,2-trichloro-1,2,2-trifluoroethane, 1,2-dichloro-1,1,2,2-tetrafluoroethane, dimethylsulfoxide, 1,1-dichloro-1,2,2,3,3-pentafluoropropane (HCFC225), ZEORORA H, HFE7100, HFE7200, HFE7300, $CF_3CH_2OH$, $CF_3CF_2CH_2OH$, or $(CF_3)_2CHOH$. These may be used singly or in combinations of two or more.

When the composition includes a solvent, for example, the compound represented by Formula (1A), (1B), (1C), or (1D) may be a substance swollen by the solvent.

For example, the solvent may be a gas. That is, the composition of the present disclosure may include a mixture of the compound represented by Formula (1A), (1B), (1C), or (1D) above and a gas of the solvent.

The solvent as a gas can be preferably a hydrocarbon, more preferably a $C_{4-10}$ hydrocarbon such as butane, pentane, hexane, cyclohexane, heptane, octane, nonane, or decane, and further preferably pentane or hexane. By including a gas of such a solvent, the contact angle of the film obtained by using the composition of the present disclosure can be improved.

The composition of the present disclosure may include a thermoplastic resin such as a non-fluororesin or a fluororesin.

Examples of the non-fluororesin include a polyolefin-based resin such as polyethylene (for example, linear low-density polyethylene, low-density polyethylene, high-density polyethylene, or ultra-high molecular weight polyethylene), polypropylene including various structural isomers (syndiotactic, isotactic, and atactic structures), poly-(4-methylpentene-1), ethylene-propylene copolymer, ethylene-vinyl acetate copolymer (EVA), a chlorinated polyethylene-based resin, or a modified polyolefin, a cycloolefin resin, a polyvinyl chloride-based resin such as polyvinyl chloride, polyvinylidene chloride, a polystyrene or polystyrene derivative-based resin including various structural isomers, polyamide, polyimide, polyamideimide, polycarbonate, an ionomer, an acrylic resin such as polymethyl methacrylate (PMMA) including various structural isomers, acrylic-styrene copolymer (AS resin), acrylonitrile-butadiene-styrene copolymer (ABS resin), butadiene-styrene copolymer, ethylene-vinyl alcohol copolymer (EVOH), a polyester such as polyethylene terephthalate (PET), polybutylene terephthalate (PBT), or polycyclohexane terephthalate (PCT), polyphenylene sulfide (PPS), polyether sulfone, polyether, polyether ketone (PEK), polyether ether ketone (PEEK), polyether ketone ketone (PEKK) polyetherimide, polyacetal (POM), polyphenylene oxide, modified polyphenylene oxide, polyarylate, aromatic polyester (liquid crystal polymer), a polyurethane-based resin, epoxy resin, phenolic resin, urea resin, melamine resin, unsaturated polyester, silicone resin, polydimethyl silicone (PDMS), polyurethane, a biodegradable resin such as polylactic acid (PLA) or polycaprolactone, or a copolymer, a mixture, a polymer alloy, or the like including the above.

Examples of the fluororesin include polytetrafluoroethylene (PTFE), tetrafluoroethylene (TFE)/perfluoroalkyl vinyl ether (PAVE) copolymer [PFA], TFE/hexafluoropropylene (HFP) copolymer [FEP], ethylene [Et]/TFE copolymer [ETFE], Et/TFE/HFP copolymer, polychlorotrifluoroethylene [PCTFE], chlorotrifluoroethylene (CTFE)/TFE copolymer, Et/CTFE copolymer, polyvinylidene fluoride [PVDF], vinylidene fluoride (VDF)/TFE copolymer, and polyvinyl fluoride [PVF].

The composition of the present disclosure may include a filler.

Examples of the filler include, in addition to silica and carbon black, a metal oxide such as calcium oxide, titanium oxide, aluminum oxide, or magnesium oxide; a metal hydroxide such as magnesium hydroxide, aluminum hydroxide, or calcium hydroxide; a carbonate such as magnesium carbonate, aluminum carbonate, calcium carbonate, or barium carbonate; a silicate such as magnesium silicate, calcium silicate, sodium silicate, or aluminum silicate; a

sulfate such as aluminum sulfate, calcium sulfate, or barium sulfate; synthetic hydrotalcite; a metal sulfide such as molybdenum disulfide, iron sulfide, or copper sulfide; diatomaceous earth, asbestos, lithopone (zinc sulfide/barium sulfide), graphite, carbon fluoride, calcium fluoride, coke, a fine quartz powder, talc, a mica powder, wollastonite, a carbon fiber, an aramid fiber, various whiskers, a glass fiber, an organic reinforcing agent, an organic filler, polytetrafluoroethylene, mica, celite, and clay. These may be used singly or as a mixture of two or more.

The compounds and the composition of the present disclosure can be used in a wide range of applications, such as a compatibilizer, an adhesive, a surface-treating agent (for example, water repellent), a highly heat-resistant material, an adsorptive separation material, and a material for a formed article.

The compounds and the composition of the present disclosure can each be formed into a formed article. Therefore, the disclosure also provides a formed article formed from any of the compounds and the composition of the disclosure.

The method for forming the compounds and the composition of the present disclosure is not limited, and may be a general forming method such as extrusion forming, injection molding, press forming, vacuum forming, transfer molding, casting, or the like.

The shape of the resulting formed article is not limited, and for example, may be any desired shape such as block-like, sheet-like, film-like, rod-like, or various other shapes depending on the applications.

For example, when obtaining a film, the pillararene of the present disclosure can be dissolved in a good solvent, cast on a substrate, and heated to the boiling point of the solvent or higher to volatilize the solvent to form a film.

A formed article of any of the compounds or the composition of the present disclosure has high heat resistance and water repellency.

A film obtained by casting any of the compounds or the composition of the present disclosure can be treated with a gas of a hydrocarbon, preferably a $C_{4-10}$ hydrocarbon such as butane, pentane, hexane, cyclohexane, heptane, octane, nonane, or decane, more preferably pentane or hexane to improve the water contact angle.

When using any of the compounds or the composition of the present disclosure as a surface-treating agent, various substrates can be treated. Therefore, the disclosure provides a material including a substrate treated with any of the compounds and/or the composition of the disclosure.

A substrate that can be treated with any of the compounds and the composition of the present disclosure can be composed of, for example, glass, a resin (which, for example, may be a natural or synthetic resin, such as a common plastic material), a metal, a ceramic, a semiconductor (silicon, germanium, or the like), a fiber (woven fabric, nonwoven fabric, or the like), fur, leather, a wood, a ceramic material, a stone material, a building member, a sanitary material, an adsorptive separation material, or any suitable material.

A substrate treated with any of the compounds or the composition of the present disclosure has high heat resistance and water repellency.

The present disclosure provides a liquid-repellent composition including a pillararene represented by Formula (1E) below and a hydrocarbon compound:

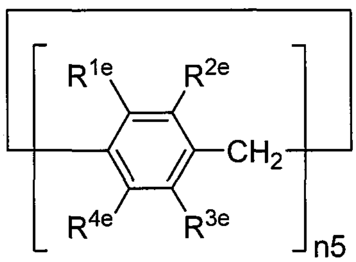

wherein

$R^{1e}$, $R^{2e}$, $R^{3e}$, and $R^{4e}$ are each independently a hydrogen atom or an organic group, and
n5 is 5 or 6.

The formed article of the liquid-repellent composition has high water repellency.

The organic group is an organic group having preferably 1 to 30 carbon atoms, and more preferably 1 to 20 carbon atoms.

The liquid-repellent composition may further include a solvent.

Examples of the solvent include the same solvents as described for the composition including the compound represented by Formula (1A), (1B), (1C), or (1D) above. The solvent included in the liquid-repellent composition can be a $C_{4-10}$ hydrocarbon such as butane, pentane, hexane, cyclohexane, heptane, octane, nonane, or decane, and further

preferably pentane or hexane.

The hydrocarbon compound included in the liquid-repellent composition can be preferably a $C_{4-10}$ hydrocarbon such as butane, pentane, hexane, cyclohexane, heptane, octane, nonane, or decane, and further preferably pentane or hexane.

The hydrocarbon compound included in the liquid-repellent composition may be the same as or different from the solvent.

In the liquid-repellent composition, the pillararene represented by Formula (1E) and the hydrocarbon compound may be included as a so-called inclusion, which is what is obtained by including the hydrocarbon compound in the pillararene represented by Formula (1E), or for example, may be a so-called surface adhered substance, which is what is obtained by adhering the hydrocarbon compound to the pillararene.

When the liquid-repellent composition includes a solvent, for example, the pillararene represented by Formula (1E) may be a substance swollen by the solvent.

The inclusion and the surface adhered substance can be obtained by treating the pillararene represented by Formula (1E) with a gas of a hydrocarbon compound.

The compounds of the present disclosure have been described in detail above. The methods for producing the compounds of the present disclosure, and the like are not limited to those given as examples above.

Examples

The compounds of the present disclosure will be more specifically described through the following Examples, but the present disclosure is not limited to these Examples.

Synthesis Example 1

Dehydrated dimethylformamide (30 mL) and dehydrated tetrahydrofuran (30 mL) were added to pillar[5]arene in a hydroquinone form (1.30 g, 2.13 mmol), and then NaH (1.33 g, 66.7 mmol) was added. 1-Tosyloxy-4,4,4-trifluorobutane (12.1 g, 42.9 mmol) was added, and then the reaction was carried out at 60°C for 72 hours. The temperature was returned to room temperature, and then methanol was added to quench the reaction. After concentration, separation was carried out with dichloromethane and saturated brine, and $Na_2SO_4$ was added to the chloroform layer for drying. $Na_2SO_4$ was removed by filtration, and concentration was carried out. Purification by silica column chromatography (hexane:dichloromethane = 2:1) gave the following substituted pillar[5]arene (yield of 15%).

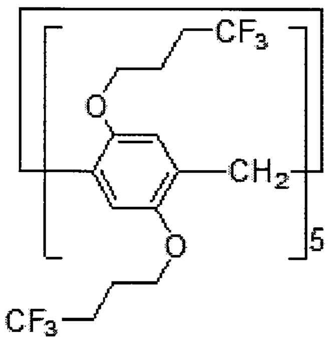

$CF_3$[5]: 1H NMR (400 MHz, CDCl3) δ 6.75 (s, 10H), 3.89 (t, J = 10.8 Hz, 20H), 3.74 (s, 10H), 2.29-2.41 (m, 20H), 2.01-2.08 (m, 20H)

Synthesis Example 2

The following substituted pillar[5]arene (yield of 13%) was obtained in the same manner as in Synthesis Example 1 except that 4,4,5,5,5-pentafluoropentyl-p-toluenesulfonate (14.3 g, 42.9 mmol) was used instead of 1-tosyloxy-4,4,4-trifluorobutane.

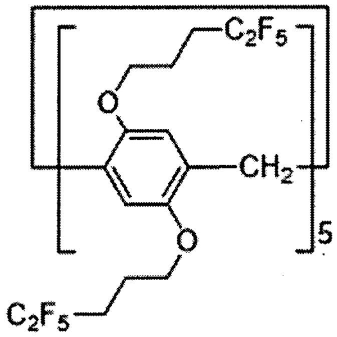

$C_2F_5$[5]: 1H NMR (400 MHz, CDCl3) δ 6.76 (s, 10H), 3.75-4.03 (m, 20H), 3.73 (s, 10H), 2.25-2.34 (m, 20H), 2.08-2.11 (m, 20H)

Synthesis Example 3

The following substituted pillar[5]arene (yield of 5.3%) was obtained in the same manner as in Synthesis Example 1 except that 8-[(6-bromohexyl)oxy]-1,1,1,2,2,3,3,4,4,5,5,6,6-tridecafluoro-octane (22.6 g, 42.9 mmol) was used instead of 1-tosyloxy-4,4,4-trifluorobutane.

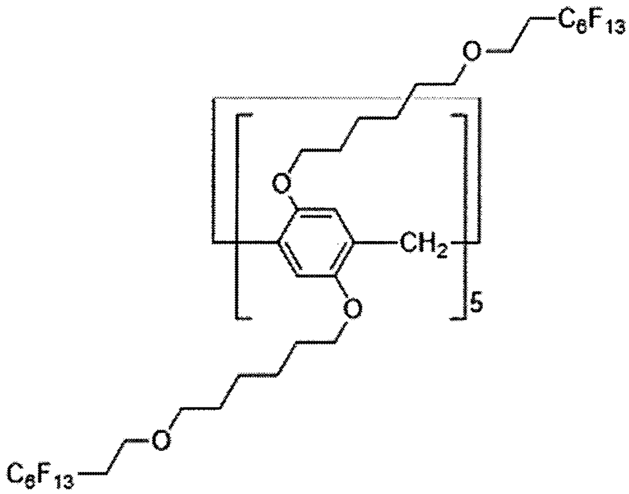

$C_6F_{13}$[5]: 1H NMR (400 MHz, CDCl3) δ 6.86 (bs, 10H), 3.24-4.21 (m, 89H), 2.38 (bs, 22H), 1.51-2.10 (m, 31H), 1.45 (bs, 28H)

Synthesis Example 4

The following substituted pillar [6] arene (yield of 24%) was obtained in the same manner as in Synthesis Example 1 except that pillar[6]arene in a hydroquinone form (1.30 g, 1.77 mmol), was used instead of pillar[5]arene in a hydroquinone form, and 4,4,5,5,5-pentafluoropentyl-p-toluenesulfonate (14.3 g, 42.9 mmol) was used instead of 1-tosyloxy-4,4,4-trifluorobutane.

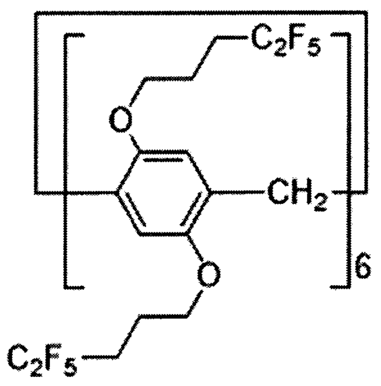

$C_2F_5$[6]: 1H NMR (400 MHz, CDCl3) δ 6.66 (s, 12H), 3.78-3.82 (m, 36H), 2.14-2.27 (m, 24H), 1.93-2.04 (m, 24H)

Synthesis Example 5

Paraformaldehyde (0.31 g, 10 mmol) was added to a solution of 1,4-dipentyloxybenzene (1.66 g, 10 mmol) in 1,2-dichloroethane (20 mL) in a nitrogen atmosphere. Next, boron trifluoride diethyl etherate (1.25 mL, 10 mmol) was added, and the resulting mixture was stirred at a temperature of 30°C for 3 hours. The solution was poured into methanol, and the precipitate was collected by filtration. The precipitate was dissolved in trichloromethane, and the undissolved portion was removed by filtration. Trichloromethane was removed under reduced pressure, and the target compound (0.413 g, yield of 23.2%) was crystallized with acetone.

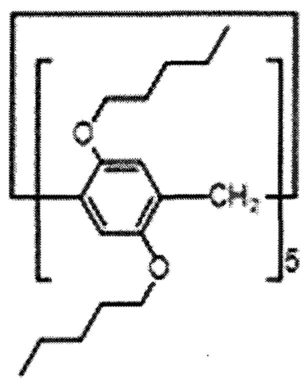

1H NMR (CDCl3, 400 MHz, TMS) δ 6.72 (s, 10H), 3.82 (q, J=7.1 Hz, 20H), 3.76 (s, 10H), 1.25 (t, J = 7.1 Hz, 30H) 13C NMR (CDCl3, 100 MHz, TMS) δ 149.8, 128.5, 115.1, 63.8, 29.8, 15.0

Synthesis Example 6

Pillar[6]arene in a hydroquinone form (0.582 g, 0.795 mmol), was dissolved in acetonitrile (60 mL). Next, pentafluorobenzyl bromide (5.00 g, 19.2 mmol)) was added, and further, $K_2CO_3$ (1.65 g, 11.9 mmol) and KI (0.319 g, 1.92 mmol) were added. The resulting mixture was heated to 60°C and allowed to react for 72 hours. The target product was isolated by silica gel chromatography (dichloromethane:hexane = 20 to 25:80 to 75). $^1$H NMR and HPLC demonstrated that the target compound was obtained.
1H NMR (CDCl3, 400 MHz, TMS) δ 6.71 (s, 12H), 4.80 (s, 24H), 3.70 (s, 12H)

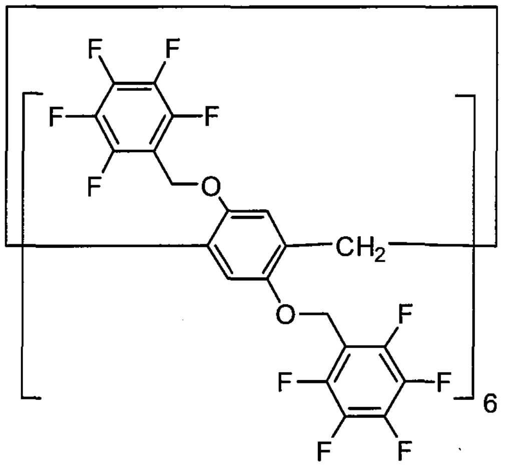

Synthesis Example 7

Pillar[5]arene in a hydroquinone form (0.305 g, 0.5 mmol), was dissolved in dimethylsulfoxide (5 mL). After that, stearyl isocyanate (1.478 g, 5 mmol) was added, and 1 drop of dibutyltin laurate as a catalyst was added. The resulting mixture was heated to 40°C and allowed to react for 6 hours. The reaction solution and the precipitate were cleaned with hexane and water in presented order to obtain the target compound. FT-IR demonstrated that the target compound was obtained, by disappearance of the peak derived from pillararene in a hydroquinone form, that was broad around 3000 to 3500 $cm^{-1}$, disappearance of the C=O peak derived from stearyl isocyanate at 2266 $cm^{-1}$, and generation of the C=O peak derived from the urethane bond around 1708 $cm^{-1}$.

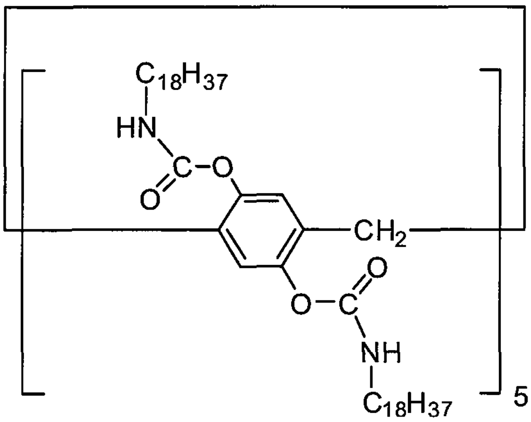

<SP value and miscibility>

(SP value)

The SP values of side chains of the pillararenes of Synthesis Examples 1 to 4 were calculated by the Fedors method described above. Results thereof are shown in the following table.

(Miscibility)

As shown in the following table, the SP values of the side chain structures of Synthesis Examples 1 to 4 were in the range of 6.9 to 7.4. On the other hand, for example, when a fluororesin having an SP value of 5.9 is selected, it is considered that the pillararene compound and the fluororesin have good miscibility because the SP values of the both are close to each other.

[Table 1]

| | Side chain structure | SP value |
|---|---|---|
| Synthesis Example 1 | $-O-(CH_2)_3-CF_3$ | 7.0 |
| Synthesis Example 2 | $-O-(CH_2)_3-C_2F_5$ | 6.9 |
| Synthesis Example 3 | $-O-(CH_2)_6-O-(CH_2)_2-C_6F_{13}$ | 7.4 |
| Synthesis Example 4 | $-O-(CH_2)_3-C2F_5$ | 6.9 |

(SP value)

When the side chain structure is a long-chain alkyloxy group: $-O-C_{17}H_{35}$, $-O-C_{20}H_{41}$, $-O-C_{26}H_{53}$, or $-O-C_{30}H_{61}$ (all linear), the SP value was calculated by the Fedors method described above. Results thereof are shown in the following table.

(Miscibility)

As shown in the following table, the SP values of the side chain structures of Reference Examples 1 to 4 were 8.4 to 8.5. On the other hand, for example, when a fluororesin having an SP value of 8.1 is selected, it is considered that the pillararene compound and the fluororesin have good miscibility because the SP values of both are close to each other.

[Table 2]

| | Side chain structure | SP value |
|---|---|---|
| Reference Example 1 | $-O-C_{17}H_{35}$ | 8.4 |
| Reference Example 2 | $-O-C_{20}H_{41}$ | 8.4 |
| Reference Example 3 | $-O-C_{26}H_{53}$ | 8.4 |
| Reference Example 4 | $-O-C_{30}H_{61}$ | 8.5 |

<Film formation>

(Formation of cast film)

The pillararenes obtained in Synthesis Examples 1, 2, 4, and 5 above were dissolved in dichloromethane. The obtained solutions were cast on glass substrates, respectively, dichloromethane was volatilized at room temperature, and then the resulting solids were heated to 160°C and melted. Next, the resulting melts were cooled at room temperature to obtain cast films of the pillararenes.

(Post-treatment)

A sample bottle containing a solvent and the cast films obtained above were added, the cast, films were exposed to solvent vapor for 17 hours, and then air-dried in a fume hood for 2 hours to obtain solvent-treated films. The combinations of cast films and solvents are as follows.

Cast film of Synthesis Example 2: pentane, hexane, heptane, octane
Cast film of Synthesis Example 4: cyclohexane
Cast film of Synthesis Example 5: pentane, hexane

Each of the air-dried samples was dissolved in deuterated chloroform, and the integrated values of the $^1H$ NMR peaks of the pillararene and the alkane moiety demonstrated that the solvent(s) was(were) included in the pillararene.

(Measurement of contact angle)

The contact angles of the cast films obtained from the compounds of Synthesis Examples 1, 2, 4, and 5 and the solvent-treated films obtained above were measured before and after the exposure. The contact angle was measured by using water with a fully automatic compact contact angle meter (P200A-HSM, Meiwafosis Co., Ltd.). Results thereof are shown in the following table.

[Table 3]

| | Initial contact angle (°) | Contact angle after solvent vapor treatment (°) | | | | |
|---|---|---|---|---|---|---|
| | | Pentane | Hexane | Cyclohexane | Heptane | Octane |
| Synthesis Example 1 | 99 | - | - | - | - | - |
| Synthesis Example 2 | 101 | 121 | 113 | - | 101 | 102 |
| Synthesis Example 4 | 108 | - | - | 110 | - | - |
| Synthesis Example 5 | 97 | 102 | 106 | - | - | - |

The above results demonstrated that the films of the pillararenes of Synthesis Examples 1, 2, 4, and 5 had good water repellency. In particular, these demonstrated that the films had much better water repellency when treated with solvent vapor.

<Melting point>

The melting points of the cast films obtained from the compounds of Synthesis Examples 1, 2, 4, and 5 were measured with DSC7020 (Hitachi High-Tech Science Co., Tokyo, Japan). Results thereof are shown in the following table.

[Table 4]

| | Melting point (°) |
|---|---|
| Synthesis Example 1 | 148 |
| Synthesis Example 2 | 117 |
| Synthesis Example 4 | 87 |
| Synthesis Example 5 | 83 |

The above results demonstrated that the pillararenes of Synthesis Examples 1, 2, and 4, particularly Synthesis Examples 1 and 2, had a high melting point and excellent heat resistance. When any of the compounds is used as an additive to a resin, the high melting point of the compound can suppress softening of the resin. Therefore, the pillararenes of the present disclosure can be used in a wide range of applications such as an application requiring heat resistance.

Industrial Applicability

The compounds of the present disclosure can be used in a wide range of applications, such as a compatibilizer, an adhesive, a surface-treating agent, a highly heat-resistant material, an adsorptive separation material, and a material for a formed article.

## Claims

**1.** A compound represented by Formula (1A) below:

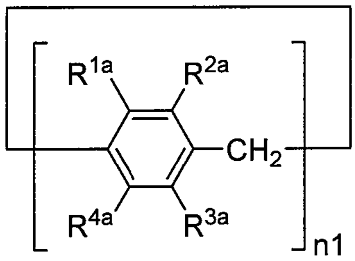

wherein

$R^{1a}$ is each independently at each occurrence a hydrogen atom or an organic group,
$R^{2a}$ is each independently at each occurrence a hydrogen atom or an organic group,
$R^{3a}$ is each independently at each occurrence a hydrogen atom or an organic group,
$R^{4a}$ is each independently at each occurrence a hydrogen atom or an organic group,
provided that at least one of $R^{1a}$, $R^{2a}$, $R^{3a}$, and $R^{4a}$ is an organic group having 2 or more carbon atoms and having a fluoroalkyl group or a fluorophenyl group, and
n1 is an integer of 4 to 20.

**2.** The compound according to claim 1, wherein

$R^{1a}$ and $R^{3a}$ are each independently an organic group,
provided that at least one of $R^{1a}$ and $R^{3a}$ is an organic group having 2 or more carbon atoms and having a fluoroalkyl group or a fluorophenyl group, or
$R^{2a}$ and $R^{4a}$ are each independently an organic group,
provided that at least one of $R^{2a}$ and $R^{4a}$ is an organic group having 2 or more carbon atoms and having a fluoroalkyl group or a fluorophenyl group.

**3.** The compound according to claim 1 or 2, wherein

$R^{1a}$ and $R^{3a}$ are each independently an organic group having 2 or more carbon atoms and having a fluoroalkyl group or a fluorophenyl group, or
$R^{2a}$ and $R^{4a}$ are each independently an organic group having 2 or more carbon atoms and having a fluoroalkyl group or a fluorophenyl group.

**4.** The compound according to any one of claims 1 to 3, wherein

$R^{2a}$ and $R^{4a}$ are each a hydrogen atom, or
$R^{1a}$ and $R^{3a}$ are each a hydrogen atom.

**5.** The compound according to any one of claims 1 to 4, wherein n1 is an integer of 4 to 6.

**6.** The compound according to any one of claims 1 to 5, wherein

the organic group having 2 or more carbon atoms and having a fluoroalkyl group is $-(O_{p1}-R^{11a}{}_{q1})-Rf^a$,
$R^{11a}$ is each independently at each occurrence a $C_{1-10}$ alkylene group or -CONH-,
$Rf^a$ is a $C_{1-10}$ fluoroalkyl group,
p1 is an integer of 0 to 2,
q1 is an integer of 0 to 3, and
in $(O_{p1}-R^{11a}{}_{q1})$, occurrence order of O and $R^{11a}$ is not limited.

**7.** The compound according to any one of claims 1 to 6, wherein

the organic group having 2 or more carbon atoms and having a fluoroalkyl group is $-O-R^{11a}-(O-R^{11a})_{r1}-Rf^a$,
$R^{11a}$ is each independently a $C_{1-10}$ alkylene group or -CONH-,
$Rf^a$ is a $C_{1-10}$ fluoroalkyl group, and
r1 is 0 or 1.

**8.** The compound according to any one of claims 1 to 7, wherein the fluoroalkyl group is a perfluoroalkyl group.

**9.** The compound according to any one of claims 1 to 8, wherein

$R^{1a}$ and $R^{3a}$ are each $-O-R^{11a}-(O-R^{11a})_{r1}-Rf^{a}$, and $R^{2a}$ and $R^{4a}$ are each a hydrogen atom, or
$R^{2a}$ and $R^{4a}$ are each $-O-R^{11a}-(O-R^{11a})_{r1}-Rf^{a}$, and $R^{1a}$ and $R^{3a}$ are each a hydrogen atom,
$R^{11a}$ is each independently a $C_{1-10}$ alkylene group or -CONH-,
$Rf^{a}$ is a $C_{1-10}$ perfluoroalkyl group,
r1 is 0 or 1, and
n1 is an integer of 4 to 6.

**10.** The compound according to any one of claims 1 to 5, wherein
the organic group having 2 or more carbon atoms and having a fluorophenyl group is a group represented by the following formula:

$$-(O_{p3}-R^{11p}{}_{q3})-Rf^{p}$$

wherein

$R^{11p}$ is each independently at each occurrence a $C_{1-10}$ alkylene group,
$Rf^{p}$ is a phenyl group substituted by 1 to 5 fluorine,
p3 is an integer of 0 to 2,
q3 is an integer of 0 to 3, and
in $(O_{p3}-R^{11p}{}_{q3})$, occurrence order of O and $R^{11p}$ is not limited.

**11.** The compound according to any one of claims 1 to 5,
wherein

the organic group having 2 or more carbon atoms and having a fluorophenyl group is a group represented by the following formula:

$$-O-R^{11p}-Rf^{p}$$

wherein
$R^{11p}$ is each independently at each occurrence a $C_{1-10}$ alkylene group, and
RfP is a phenyl group substituted with 1 to 5 fluorine atoms.

**12.** The compound according to any one of claims 1 to 5, wherein

$R^{1a}$ and $R^{3a}$ are each $-O-R^{11p}-Rf^{p}$, and $R^{2a}$ and $R^{4a}$ are each a hydrogen atom, or
$R^{2a}$ and $R^{4a}$ are $-O-R^{11p}-Rf^{p}$, and $R^{1a}$ and $R^{3a}$ are each a hydrogen atom,
$R^{11p}$ is each independently a $C_{1-6}$ alkylene group,
RfP is a phenyl group substituted with 1 to 5 fluorine atoms, and
n1 is an integer of 4 to 6.

**13.** The compound according to any one of claims 1 to 12, wherein when the compound is formed into a film, a static contact angle of the film with water is 80° or more.

**14.** A compound represented by Formula (1B) below:

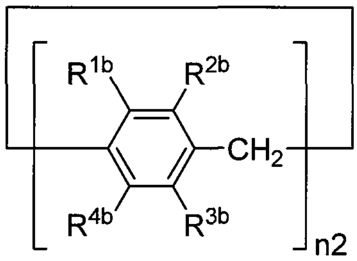

wherein

$R^{1b}$ is each independently at each occurrence a hydrogen atom or an organic group,
$R^{2b}$ is each independently at each occurrence a hydrogen atom or an organic group,
$R^{3b}$ is each independently at each occurrence a hydrogen atom or an organic group, and
$R^{4b}$ is each independently at each occurrence a hydrogen atom or an organic group,
provided that at least one of $R^{1b}$, $R^{2b}$, $R^{3b}$, and $R^{4b}$ is an organic group having a fluorine atom, and the organic group having a fluorine atom has an SP value of 8.0 or less, and n2 is an integer of 4 to 20.

**15.** The compound according to claim 14, wherein

$R^{1b}$ and $R^{3b}$ are each independently an organic group,
provided that at least one of $R^{1b}$ and $R^{3b}$ is an organic group having a fluorine atom and having an SP value of 8.0 or less, or
$R^{2b}$ and $R^{4b}$ are each independently an organic group,
provided that at least one of $R^{2b}$ and $R^{4b}$ is an organic group having a fluorine atom and having an SP value of 8.0 or less.

**16.** The compound according to claim 14 or 15, wherein

$R^{1b}$ and $R^{3b}$ are each independently an organic group having a fluorine atom and having an SP value of 8.0 or less, or
$R^{2b}$ and $R^{4b}$ are each independently an organic group having a fluorine atom and having an SP value of 8.0 or less.

**17.** The compound according to any one of claims 14 to 16, wherein

$R^{2b}$ and $R^{4b}$ are each a hydrogen atom, or
$R^{1b}$ and $R^{3b}$ are each a hydrogen atom.

**18.** The compound according to any one of claims 14 to 17, wherein n2 is an integer of 4 to 6.

**19.** The compound according to any one of claims 14 to 18, wherein the SP value of the organic group having a fluorine atom is 4.0 to 7.5.

**20.** The compound according to any one of claims 14 to 19, wherein the organic group having a fluorine atom has a $C_{1-10}$ fluoroalkyl group.

**21.** The compound according to claim 20, wherein the $C_{1-10}$ fluoroalkyl group is a $C_{1-10}$ perfluoroalkyl group.

**22.** The compound according to any one of claims 14 to 21, wherein

$R^{1b}$ and $R^{3b}$ are each $-O-R^{11b}-(O-R^{11b})_{r2}-Rf^b$, and $R^{2b}$ and $R^{4b}$ are each a hydrogen atom, or
$R^{2b}$ and $R^{4b}$ are each $-O-R^{11b}-(O-R^{11b})_{r2}-Rf^b$, and $R^{1b}$ and $R^{3b}$ are each a hydrogen atom,
$R^{11b}$ is each independently a $C_{1-10}$ alkylene group,
$Rf^b$ is a $C_{1-10}$ perfluoroalkyl group,
r2 is 0 or 1, and
n2 is an integer of 4 to 6.

**23.** The compound according to any one of claims 14 to 22, wherein when the compound is formed into a film, a static contact angle of the film with water is 80° or more.

**24.** A compound represented by Formula (1C) below:

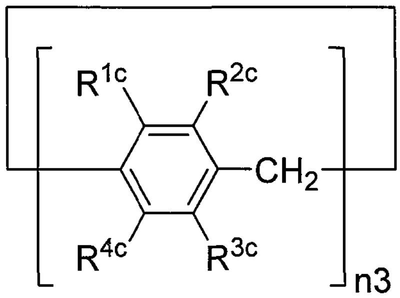

wherein

$R^{1c}$ is each independently at each occurrence a hydrogen atom or an organic group,
$R^{2c}$ is each independently at each occurrence a hydrogen atom or an organic group,
$R^{3c}$ is each independently at each occurrence a hydrogen atom or an organic group,
$R^{4c}$ is each independently at each occurrence a hydrogen atom or an organic group,
provided that at least one of $R^{1c}$, $R^{2c}$, $R^{3c}$, and $R^{4c}$ is an organic group having a long-chain alkyl group having 17 or more carbon atoms, and
n3 is an integer of 4 to 20.

**25.** The compound according to claim 24, wherein

$R^{1c}$ and $R^{3c}$ are each independently an organic group,
provided that at least one of $R^{1c}$ and $R^{3c}$ is an organic group having a long-chain alkyl group having 17 or more carbon atoms, or
$R^{2c}$ and $R^{4c}$ are each independently an organic group,
provided that at least one of $R^{2c}$ and $R^{4c}$ is an organic group having a long-chain alkyl group having 17 or more carbon atoms.

**26.** The compound according to claim 24 or 25, wherein

$R^{1c}$ and $R^{3c}$ are each independently an organic group having a long-chain alkyl group having 17 or more carbon atoms, and
$R^{2c}$ and $R^{4c}$ are each independently an organic group having a long-chain alkyl group having 17 or more carbon atoms.

**27.** The compound according to any one of claims 24 to 26, wherein the number of carbon atoms in the long-chain alkyl group is 17 to 30.

**28.** The compound according to any one of claims 24 to 27, wherein

$R^{1c}$ and $R^{3c}$ are each independently -O-$R^{12c}$, or
$R^{2c}$ and $R^{4c}$ are each independently -O-$R^{12c}$, and
$R^{12c}$ is a $C_{17-30}$ alkyl group.

**29.** The compound according to any one of claims 24 to 28, wherein

$R^{2c}$ and $R^{4c}$ are each a hydrogen atom, or
$R^{1c}$ and $R^{3c}$ are each a hydrogen atom.

**30.** The compound according to any one of claims 24 to 29, wherein n3 is an integer of 4 to 6.

**31.** The compound according to any one of claims 24 to 30, wherein

$R^{1c}$ and $R^{3c}$ are each -O-$R^{12c}$, and $R^{2c}$ and $R^{4c}$ are each a hydrogen atom, or
$R^{2c}$ and $R^{4c}$ are each -O-$R^{12c}$, and $R^{1c}$ and $R^{3c}$ is each a hydrogen atom,
$R^{12c}$ is a $C_{17-30}$ alkyl group, and
n3 is an integer of 4 to 6.

**32.** The compound according to any one of claims 24 to 31, wherein when the compound is formed into a film, a static contact angle of the film with water is 80° or more.

**33.** A compound represented by Formula (1D) below:

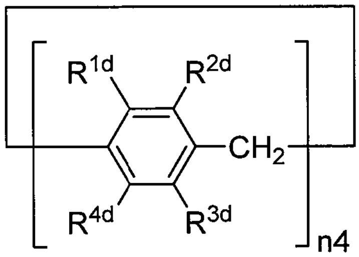

wherein

$R^{1d}$ is each independently at each occurrence a hydrogen atom or an organic group,
$R^{2d}$ is each independently at each occurrence a hydrogen atom or an organic group,
$R^{3d}$ is each independently at each occurrence a hydrogen atom or an organic group, and
$R^{4d}$ is each independently at each occurrence a hydrogen atom or an organic group,
provided that at least one of $R^{1d}$, $R^{2d}$, $R^{3d}$, and $R^{4d}$ is an organic group having an alkyl group, and the alkyl group has an SP value of 8.2 or more, and
n4 is an integer of 4 to 20.

**34.** The compound according to claim 33, wherein

$R^{1d}$ and $R^{3d}$ are each independently an organic group,
provided that at least one of $R^{1d}$ and $R^{3d}$ is an organic group having an alkyl group having an SP value of 8.2 or more, or
$R^{2d}$ and $R^{4d}$ are each independently an organic group,
provided that at least one of $R^{2d}$ and $R^{4d}$ is an organic group having an alkyl group having an SP value of 8.2 or more.

**35.** The compound according to claim 33 or 34, wherein

$R^{1d}$ and $R^{3d}$ are each independently an organic group having an alkyl group having an SP value of 8.2 or more, or
$R^{2d}$ and $R^{4d}$ are each independently an organic group having an alkyl group having an SP value of 8.2 or more.

**36.** The compound according to any one of claims 33 to 35, wherein

$R^{2d}$ and $R^{4d}$ are each a hydrogen atom, or
$R^{1d}$ and $R^{3d}$ are each a hydrogen atom.

**37.** The compound according to any one of claims 33 to 36, wherein n4 is an integer of 4 to 6.

**38.** The compound according to any one of claims 33 to 37, wherein the SP value of the alkyl group is 8.2 to 8.4.

**39.** The compound according to any one of claims 33 to 38, wherein

$R^{1d}$ and $R^{3d}$ are each -O-$R^{12d}$, and $R^{2d}$ and $R^{4d}$ are each a hydrogen atom, or

$R^{2d}$ and $R^{4d}$ are each -O-$R^{12d}$, and $R^{1d}$ and $R^{3d}$ is each a hydrogen atom,
$R^{12d}$ is a $C_{17-30}$ alkyl group, and
n4 is an integer of 4 to 6.

**40.** The compound according to any one of claims 33 to 39, wherein when the compound is formed into a film, a static contact angle of the film with water is 80° or more.

**41.** A composition comprising at least one of the compound according to any one of claims 1 to 40.

**42.** A formed article of the compound according to any one of claims 1 to 40.

**43.** A formed article formed from the composition according to claim 41.

**44.** The formed article according to claim 42 or 43, wherein the formed article is a film.

**45.** A material comprising a substrate treated with the compound according to any one of claims 1 to 40 or the composition according to claim 41.

**46.** A liquid-repellent composition comprising a pillararene represented by Formula (1E) below and a hydrocarbon compound:

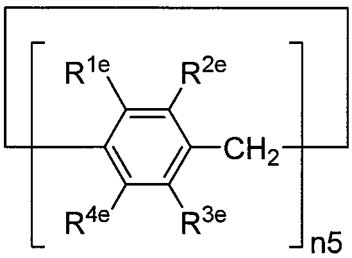

wherein

$R^{1e}$, $R^{2e}$, $R^{3e}$, and $R^{4e}$ are each independently a hydrogen atom or an organic group, and
n5 is 5 or 6.

**47.** The liquid-repellent composition according to claim 46, wherein the organic group is an organic group having 1 to 30 carbon atoms.

**48.** The liquid-repellent composition of claim 46 or 47, wherein the liquid-repellent composition further comprises a solvent.

**INTERNATIONAL SEARCH REPORT**

International application No.
**PCT/JP2022/024222**

**A. CLASSIFICATION OF SUBJECT MATTER**

***C07C 43/205***(2006.01)i; ***C08G 8/28***(2006.01)i; ***C08L 61/12***(2006.01)i; ***C09K 3/18***(2006.01)i
FI: C07C43/205 C; C08G8/28 Z; C08L61/12; C09K3/18 102

According to International Patent Classification (IPC) or to both national classification and IPC

**B. FIELDS SEARCHED**

Minimum documentation searched (classification system followed by classification symbols)

C07C43/205; C08G8/28; C08L61/12; C09K3/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | CN 108659215 A (FUZHOU UNIVERSITY) 16 October 2018 (2018-10-16)<br>claims, examples 1-3, paragraphs [0002]-[0004] | 1-10, 13, 41-48<br>11-12, 14-40 |
| A | JP 2015-221893 A (UNIV KANAZAWA) 10 December 2015 (2015-12-10)<br>claims, examples, etc. | 1-48 |
| A | JP 2018-39758 A (UNIV KANAZAWA) 15 March 2018 (2018-03-15)<br>claims, examples, etc. | 1-48 |
| A | 大山直樹　外４名, アミド基の導入による液晶性ピラー[n]アレーンの創成, 日本化学会春季年会講演予稿集, vol. 101st, 19 March 2021, A09-1vn-08<br>entire text, (OYAMA, Naoki et al. Construction of Liquid Crystalline Pillar[n]arenes by Introducing Amide Groups.), non-official translation (Lecture preprints of the CSJ Annual Meeting) | 1-48 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 August 2022** | **06 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/024222**

**C. DOCUMENTS CONSIDERED TO BE RELEVANT**

| Category* | Citation of document, with indication, where appropriate, of the relevant passages | Relevant to claim No. |
|---|---|---|
| A | 生越友樹 外２名, 柱型環状分子ピラー[n]アレーンを基にしたソフトクリスタルの創成, 日本画像学会誌, 2020, vol. 59, no. 3, 301-307<br>entire text, (OGOSHI, Tomoki et al. Construction of Soft Crystals Based on Pillar-shaped Macrocycles Pillar[n]arenes. NIHON GAZO GAKKAISHI.) | 1-48 |
| A | 生越友樹 外３名, 構造性液体Pillar[6]areneのゲスト蒸気による状態変化, 高分子学会予稿集, 2018, vol. 67, no. 2, 2F15<br>entire text (fig. 1, etc.), (OGOSHI, Tomoki et al. Polymer Preprints, Japan.), non-official translation (State change by guest vapor in structural liquid Pillar[6]arene) | 1-48 |
| A | XIAO, Wang et al. Introduction of polar or nonpolar groups at the hydroquinone units can lead to the destruction of the columnar structure of Pillar[5]arenes. Computational and Theoretical Chemistry. 2019, 1161, 1-9<br>scheme 1, etc. | 1-48 |
| A | OGOSHI, Tomoki et al. Guest Vapor-Induced State Change of Structural Liquid Pillar[6]arene. Journal of the American Chemical Society. 2019, 141(2), 785-789<br>fig. 1, etc. | 1-48 |
| P, X | ONISHI, Katsuto et al. State- and water repellency-controllable molecular glass of pillar[5]arenes with fluoroalkyl groups by guest vapors. Chemical Science. 16 March 2022, 13(14), 4082-4087<br>fig. 1, conclusion, etc. | 1-23, 41-48 |
| P, X | WANG, Zhuo et al. ortho-Functionalization of Pillar[5]arene: An Approach to Mono-ortho-Alkyl/Aryl-Substituted A1/A2-Dihydroxypillar[5]arene. Organic Letters. 28 February 2022, 24(9), 1822-1826<br>schemes 1, 2, etc. | 1-10, 13, 41-48 |

Form PCT/ISA/210 (second sheet) (January 2015)

**INTERNATIONAL SEARCH REPORT**
**Information on patent family members**

International application No.
**PCT/JP2022/024222**

| Patent document cited in search report | | | Publication date (day/month/year) | Patent family member(s) | Publication date (day/month/year) |
|---|---|---|---|---|---|
| CN | 108659215 | A | 16 October 2018 | (Family: none) | |
| JP | 2015-221893 | A | 10 December 2015 | (Family: none) | |
| JP | 2018-39758 | A | 15 March 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015221893 A


### Non-patent literature cited in the description


- **R.F. FEDORS.** *Polyme. Eng. Sic.,* 1974, vol. 14, 147